# EUROPEAN PATENT APPLICATION

(11) **EP 4 850 743 A1**
(43) Date of publication of application: **09.12.2026**
(21) Application number: 25757798.1
(22) Date of filing: 18.02.2025
(51) Int. Cl.: A61M 39/26, A61M 39/10, A61J 1/20

(54) **MALE CONNECTOR**

(30) Priority: 20.02.2024 JP 2024023946
(71) Applicant: Terumo Kabushiki Kaisha, Tokyo 151-0072 (JP)
(72) Inventor: AKIYAMA, Kazuya, Nakakoma-gun, Yamanashi 409-3853 (JP)
(74) Representative: KIPA AB
(86) International application number: PCT/JP2025/005433
(87) International publication number: WO 2025/178029

(57) **Abstract**

Provided is a male connector connectable to a female connector having an elastic valve body, the male connector including: a flow passage tubular member having an opening; a housing; an elastic opening/closing portion; a movable body having a movable body restriction portion and holding the elastic opening/closing portion; and a biasing portion including all portions capable of biasing the elastic opening/closing portion toward an axially distal side, in which the male connector changes from a first form in which the opening is closed to a second form in which the opening is opened by a connecting operation where the elastic valve body is pressed against a distal surface of the elastic opening/closing portion against a biasing force of the biasing portion to cause the elastic opening/closing portion and the movable body to move, the movable body restriction portion restricts movement of the female connector by moving radially inward through the connecting operation, and releases the restriction through a release operation that changes a form from the second form to the first form, and the biasing force becomes maximum after start of the connecting operation and before completion of the connecting operation.

## Description

### Technical Field

The present disclosure relates to a male connector.

### Background Art

A male connector connectable to a female connector having an elastic valve body is known, the male connector including: a flow passage tubular member having an opening at a portion on an axially distal side; a housing continuous with the flow passage tubular member; an elastic opening/closing portion; a movable body having a movable body restriction portion and holding the elastic opening/closing portion; and a bellows-shaped biasing portion capable of biasing the elastic opening/closing portion toward the axially distal side, in which the male connector changes from a first form in which the opening of the flow passage tubular member is closed by the elastic opening/closing portion at an initial position to a second form in which the opening of the flow passage tubular member is opened by a connecting operation where the elastic valve body is pressed against a distal surface of the elastic opening/closing portion against a biasing force of the biasing portion to cause the elastic opening/closing portion and the movable body to move toward an axially proximal side relative to the flow passage tubular member, such that the portion on the axially distal side of the flow passage tubular member protrudes from the elastic opening/closing portion toward the axially distal side upon completion of the connecting operation, and the movable body restriction portion moves radially inward by receiving a reaction force from the housing due to the connecting operation to restrict movement of the female connector toward the axially distal side relative to the elastic opening/closing portion, and moves radially outward due to a release operation for changing the form from the second form to the first form to release the restriction on the movement of the female connector (for example, see Patent Literature 1).

### Citation List

### Patent Literature

Patent Literature 1: WO 2022/149339 A1

### Summary of Invention

### Technical Problem

In the male connector as described above, since the biasing force of the bellows-shaped biasing portion increases in accordance with the connecting operation and becomes maximum upon completion of the connecting operation, a large operating force is required for the connecting operation to reach the second form.

Therefore, an object of the present disclosure is to provide a male connector that can be connected with a small operating force.

### Solution to Problem

One aspect of the present disclosure is as follows.

[1] A male connector connectable to a female connector having an elastic valve body, the male connector including:
   a flow passage tubular member defining an axial direction and a radial direction and having an opening at a portion on an axially distal side;
   a housing continuous with the flow passage tubular member;
   an elastic opening/closing portion;
   a movable body having a movable body restriction portion and holding the elastic opening/closing portion; and
   a biasing portion including all portions capable of biasing the elastic opening/closing portion toward the axially distal side,
   in which the male connector changes from a first form in which the opening of the flow passage tubular member is closed by the elastic opening/closing portion at an initial position to a second form in which the opening of the flow passage tubular member is opened by a connecting operation where the elastic valve body is pressed against a distal surface of the elastic opening/closing portion against a biasing force of the biasing portion to cause the elastic opening/closing portion and the movable body to move toward an axially proximal side relative to the flow passage tubular member, such that the portion on the axially distal side of the flow passage tubular member protrudes from the elastic opening/closing portion toward the axially distal side upon completion of the connecting operation,
   the movable body restriction portion restricts movement of the female connector toward the axially distal side relative to the elastic opening/closing portion by moving radially inward upon receiving a reaction force from the housing due to the connecting operation, and releases the restriction on the movement of the female connector by moving radially outward through a release operation that changes a form from the second form to the first form, and
   the biasing force becomes maximum after start of the connecting operation and before completion of the connecting operation.
[2] The male connector according to [1], in which the portion on the axially distal side of the flow passage tubular member has a tapered surface in which an outer peripheral surface narrows toward the axially distal side,
   in the first form, the elastic opening/closing portion is in contact with the tapered surface, and
   the biasing portion is constituted by the tapered surface.
[3] The male connector according to [2], in which the tapered surface of the flow passage tubular member extends across the opening in the axial direction.
[4] The male connector according to any one of [1] to [3], in which the movable body restriction portion has a receiving surface that receives a reaction force from the housing toward the axially distal side by being pressed against the housing radially outward by a restoring force due to elastic deformation of the movable body restriction portion, and
   the biasing portion is constituted by the receiving surface.
[5] The male connector according to any one of [1] to [4], in which the movable body restriction portion is caught on the female connector to restrict the movement of the female connector toward the axially distal side relative to the elastic opening/closing portion.
[6] The male connector according to any one of [1] to [5], in which the housing has a housing restriction portion that restricts the movement of the female connector toward the axially distal side relative to the elastic opening/closing portion by changing a form from the first form to the second form.
[7] The male connector according to [6], in which the housing restriction portion is caught on the female connector to restrict the movement of the female connector toward the axially distal side relative to the elastic opening/closing portion.
[8] The male connector according to any one of [1] to [7], configured as a medical device.
[9] A connection structure including the male connector according to any one of [1] to [8] and the female connector.
[10] The connection structure according to claim 9, in which each of the male connector and the female connector is configured as a medical device.

### Advantageous Effects of Invention

According to the present disclosure, it is possible to provide a male connector that can be connected with a small operating force.

### Brief Description of Drawings

[Fig. 1] Fig. 1 is a cross-sectional view illustrating a state before connection in a connection structure according to a first embodiment of the present disclosure.
[Fig. 2] Fig. 2 is a cross-sectional view illustrating the state of Fig. 1 at an angle different by 90°.
[Fig. 3] Fig. 3 is a cross-sectional view illustrating a state when a connecting operation is started from the state in Fig. 1.
[Fig. 4] Fig. 4 is a cross-sectional view illustrating a state when the connecting operation is further performed from the state in Fig. 3.
[Fig. 5] Fig. 5 is a cross-sectional view illustrating a state when the connecting operation is further performed from the state of Fig. 4 and completed.
[Fig. 6] Fig. 6 is a cross-sectional view illustrating the state of Fig. 5 at an angle different by 90°.
[Fig. 7] Fig. 7 is a cross-sectional view illustrating a male connector according to a second embodiment of the present disclosure.
[Fig. 8] Fig. 8 is a cross-sectional view illustrating a male connector according to a third embodiment of the present disclosure.

### Description of Embodiments

An embodiment of the present disclosure will be described in detail below with reference to the drawings.

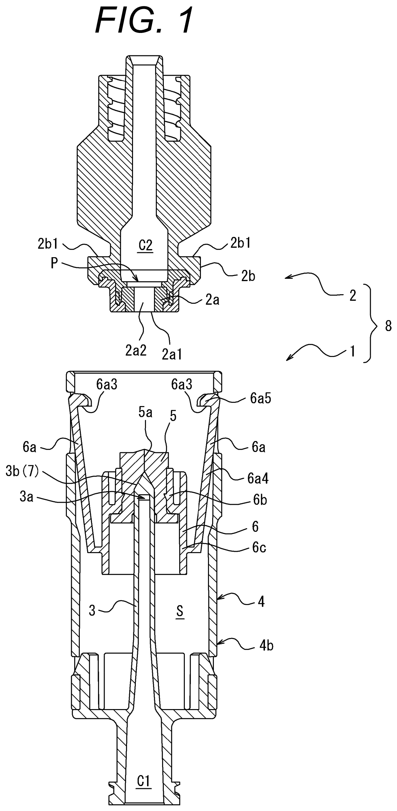

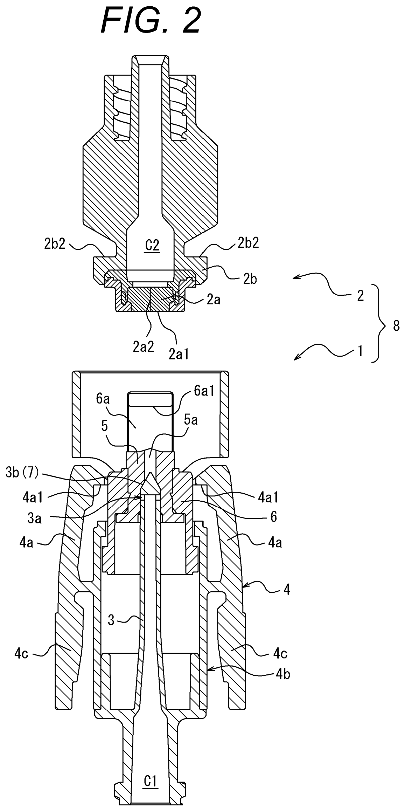

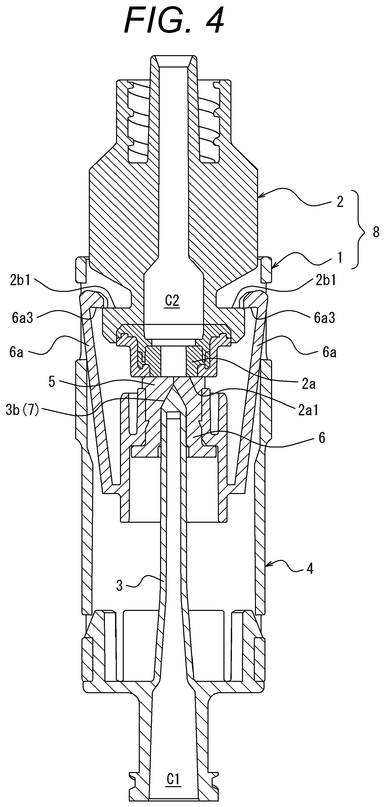

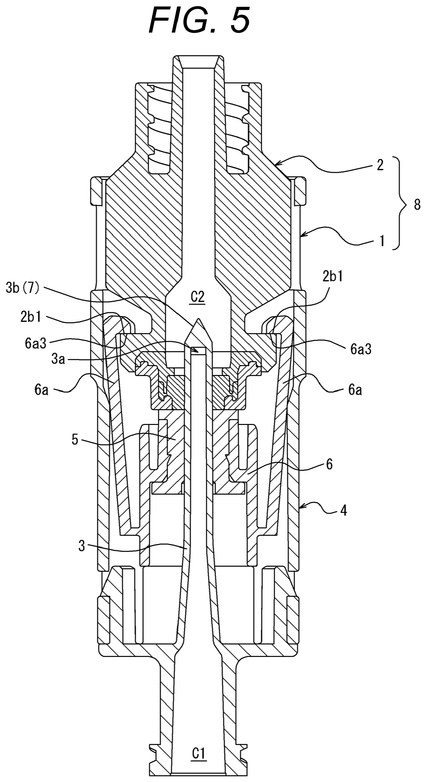

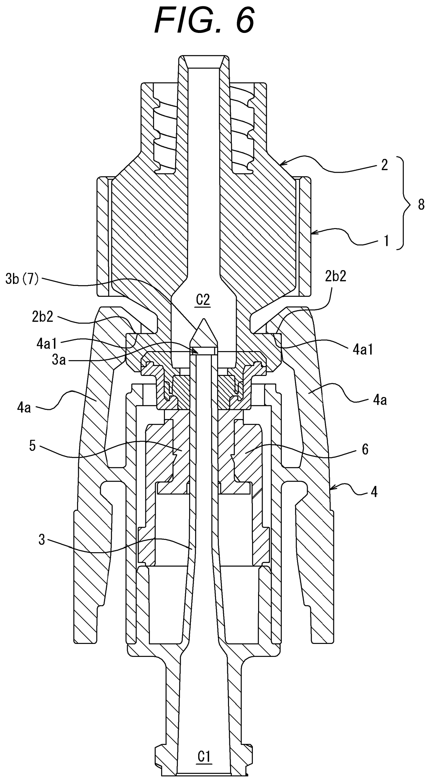

As illustrated in Figs. 1 to 6, a male connector 1 according to a first embodiment of the present disclosure is a male connector 1 connectable to a female connector 2 having an elastic valve body 2a, the male connector 1 including: a flow passage tubular member 3 defining an axial direction and a radial direction and having an opening 3a at a portion on an axially distal side; a housing 4 continuous with the flow passage tubular member 3; an elastic opening/closing portion 5; a movable body 6 having a movable body restriction portion 6a and holding the elastic opening/closing portion 5; and a biasing portion 7 including all portions capable of biasing the elastic opening/closing portion 5 toward the axially distal side (that is, capable of generating a biasing force capable of moving the elastic opening/closing portion 5 toward the axially distal side), in which the male connector 1 changes from a first form (see Figs. 1 and 2) in which the opening 3a of the flow passage tubular member 3 is closed by the elastic opening/closing portion 5 at an initial position to a second form (see Figs. 5 and 6) in which the opening 3a of the flow passage tubular member 3 is opened by a connecting operation where an elastic valve body 2a is pressed against a distal surface of the elastic opening/closing portion 5 against a biasing force of the biasing portion 7 to cause the elastic opening/closing portion 5 and the movable body 6 to move toward the axially proximal side relative to the flow passage tubular member 3, such that the portion on the axially distal side of the flow passage tubular member 3 protrudes from the elastic opening/closing portion 5 toward the axially distal side upon completion of the connecting operation, and the movable body restriction portion 6a restricts the movement of the female connector 2 toward the axially distal side relative to the elastic opening/closing portion 5 by moving radially inward upon receiving a reaction force from the housing 4 due to the connecting operation (see Fig. 4), and releases the restriction on the movement of the female connector 2 by moving radially outward through a release operation that changes the form from the second form to the first form, and the biasing force becomes maximum after the start of the connecting operation and before the completion of the connecting operation.

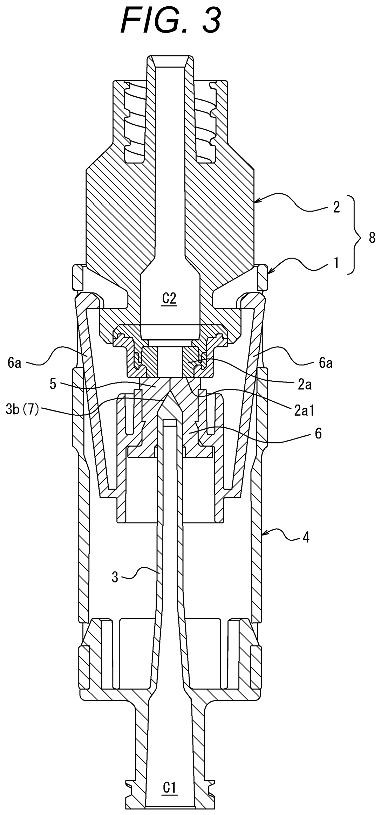

According to the above configuration, the first form in which an internal flow passage of the male connector 1 (male connector flow passage C1) is closed by the elastic opening/closing portion 5, and an internal flow passage of the female connector 2 (female connector flow passage C2) is closed by the elastic valve body 2a, is changed to the second form in which the elastic valve body 2a is brought into close contact with the elastic opening/closing portion 5 by the reaction force of the biasing portion 7 through the connecting operation while maintaining the close contact state, and thus the male connector flow passage C1 and the female connector flow passage C2 can communicate with each other through the opening 3a of the flow passage tubular member 3, and a fluid can flow. Furthermore, at the time of the release operation, the elastic opening/closing portion 5 is moved toward the axially distal side from the movable body restriction portion 6a along with the movement of the female connector 2 toward the axially distal side, and thereafter, after changing from a movable body restricted state (see Figs. 4 and 5) in which the movable body restriction portion 6a restricts the movement of the female connector 2 to a movable body released state (see Figs. 1 and 3) in which the movable body restriction portion 6a releases the restriction on the movement of the female connector 2, the elastic opening/closing portion 5 can be returned to the initial position by the biasing portion 7. For this reason, for example, as in the conventional configuration in which a bellows-like spring portion is provided as described in Patent Literature 1, it is not necessary to provide a configuration in which the biasing force for returning the elastic opening/closing portion 5 to the initial position is constantly applied from the elastic member at the time of the release operation. Therefore, the biasing portion 7 including all portions capable of biasing the elastic opening/closing portion 5 toward the axially distal side can apply the maximum biasing force after the start of the connecting operation and before the completion of the connecting operation, and as a result, it is possible to avoid the need for a large operating force near the completion of the connecting operation, as in the conventional configuration. Therefore, it is possible to implement the male connector 1 that can be connected with a small operating force.

The portion on the axially distal side of the flow passage tubular member 3 has a tapered surface 3b in which an outer peripheral surface narrows toward the axially distal side up to an axially distal end thereof. In the first form, the elastic opening/closing portion 5 is in contact with the tapered surface 3b, and the biasing portion 7 is constituted by the tapered surface 3b. According to the above configuration, at the time of the connecting operation, the reaction force can be generated by the biasing force toward the axially distal side, which is applied to the elastic opening/closing portion 5 from the tapered surface 3b of the biasing portion 7 in contact with the elastic opening/closing portion 5. Furthermore, at the time of the release operation, the elastic opening/closing portion 5 can be returned to the position in the first form by the biasing force from the tapered surface 3b of the biasing portion 7. When the distal surface of the elastic opening/closing portion 5 is located closer to the axially distal side than the proximal end of the tapered surface 3b, the tapered surface 3b can bias the elastic opening/closing portion 5 toward the axially distal side by a restoring force of the elastic opening/closing portion 5 directed toward the tapered surface 3b due to the elastic deformation. Furthermore, when the distal surface of the elastic opening/closing portion 5 is located closer to the axially proximal side than the tapered surface 3b, the biasing force by the tapered surface 3b does not occur. Note that, in the present embodiment, the diameter of the portion of the outer peripheral surface of the flow passage tubular member 3 that is closer to the axially proximal side than the tapered surface 3b and on which the elastic opening/closing portion 5 can slide is constant in the axial direction, but the present disclosure is not limited thereto.

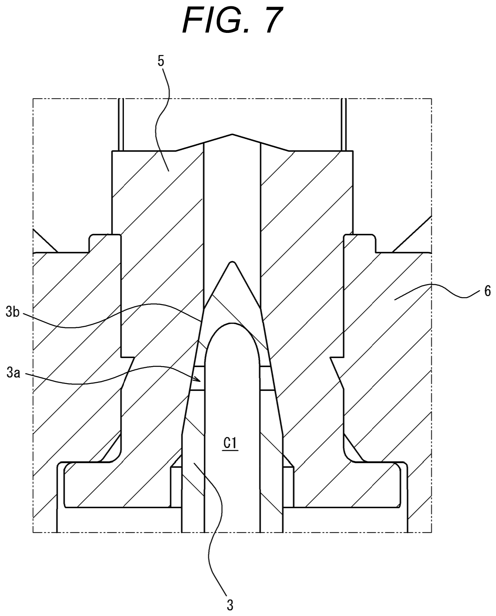

In the present embodiment, the proximal end of the tapered surface 3b is located closer to the axially distal side than the proximal end of the opening 3a, but the present disclosure is not limited thereto, and as will be described in a second embodiment illustrated in Fig. 7, the tapered surface 3b of the flow passage tubular member 3 may extend across the opening 3a in the axial direction. According to the above configuration, even when the distal surface of the elastic opening/closing portion 5 is located on the axially proximal side relative to the proximal end of the opening 3a, the biasing force can be obtained by the tapered surface 3b, such that it is easy to obtain a good reaction force at the time of the connecting operation and a good biasing force for returning to the first form at the time of the release operation.

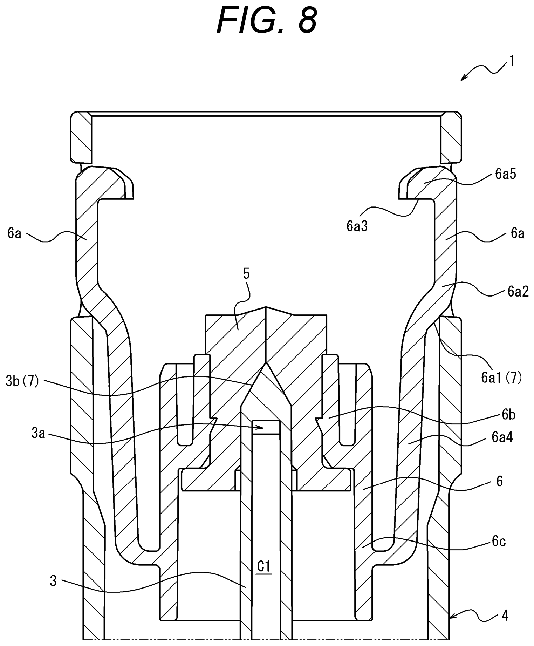

As will be described in the third embodiment illustrated in Fig. 8, the movable body restriction portion 6a may have a receiving surface 6a1 that contacts the housing 4 and is pressed against the housing 4 radially outward by a restoring force due to elastic deformation of the movable body restriction portion 6a in the first form to receive a reaction force from the housing 4 toward the axially distal side, and the biasing portion 7 may be constituted by the receiving surface 6a1. According to the above configuration, at the time of the connecting operation, the reaction force can be generated by the biasing force toward the axially distal side, which is applied to the elastic opening/closing portion 5 via the movable body restriction portion 6a from the receiving surface 6a1 in contact with the housing 4. Furthermore, at the time of the release operation, the elastic opening/closing portion 5 can be returned to the position in the first form by the biasing force from the receiving surface 6a1 of the biasing portion 7 via the movable body restriction portion 6a. In the third embodiment, the movable body restriction portion 6a has a bent portion 6a2 that bends radially inward toward the axially proximal side, and the receiving surface 6a1 extends from the bent portion 6a2 toward the axially proximal side and radially inward. When the state of the movable body 6 changes between the movable body restricted state and the movable body released state, the receiving surface 6a1 can bias the elastic opening/closing portion 5 toward the axially distal side by a restoring force directed radially outward due to the elastic deformation of the movable body restriction portion 6a. Furthermore, the receiving surface 6a1 does not generate the biasing force when the state of the movable body 6 does not change between the movable body restricted state and the movable body released state.

The movable body restriction portion 6a is caught on the female connector 2 to restrict the movement of the female connector 2 toward the axially distal side relative to the elastic opening/closing portion 5. That is, the movable body restriction portion 6a has a movable body catching portion 6a3 that is caught on a movable body caught portion 2b1 of the female connector 2. The movable body restriction portion 6a can release the catch on the female connector 2 by swinging radially outward through restoration from elastic deformation. The movable body restriction portion 6a has an arm portion 6a4 extending radially outward and toward the axially distal side, and a protrusion 6a5 protruding radially inward from the end of the arm portion 6a4 on the axially distal side and constituting the movable body catching portion 6a3. As in the third embodiment, the arm portion 6a4 may have the receiving surface 6a1 and the bent portion 6a2.

The movable body 6 has, for example, a tubular movable body base portion 6c integrally connecting a holding portion 6b and a root of the movable body restriction portion 6a located on the axially proximal side relative to the holding portion 6b. The holding portion 6b, the movable body restriction portion 6a, and the movable body base portion 6c form an integrated movable body 6. The movable body 6 has a first movable body restriction portion 6a and a second movable body restriction portion 6a, and the holding portion 6b is located between the first movable body restriction portion 6a and the second movable body restriction portion 6a, or the first movable body restriction portion 6a and the second movable body restriction portion 6a are provided so as to face each other (offset by 180°).

The elastic opening/closing portion 5 has a slit 5a through which the flow passage tubular member 3 passes when the form changes from the first form to the second form. The elastic opening/closing portion 5 is formed of, for example, rubber or elastomer. The movable body 6, the flow passage tubular member 3, and the housing 4 are formed of, for example, a resin.

The housing 4 has a housing restriction portion 4a that restricts the movement of the female connector 2 toward the axially distal side relative to the elastic opening/closing portion 5 by changing the form from the first form to the second form.

The housing restriction portion 4a is caught on the female connector 2 to restrict the movement of the female connector 2 toward the axially distal side relative to the elastic opening/closing portion 5. That is, the housing restriction portion 4a has a housing catching portion 4a1 that is caught on a housing caught portion 2b2 of the female connector 2. The housing restriction portion 4a is caught on the female connector 2 by swinging. The housing restriction portion 4a swings radially inward toward the flow passage tubular member 3 due to restoration from elastic deformation, thereby being caught on the female connector 2.

The housing 4 has a housing base portion 4b that is continuous with the flow passage tubular member 3 and forms a hollow portion S in which the flow passage tubular member 3 is located, a housing restriction portion 4a that has a housing catching portion 4a1 on the axially distal side and is swingably continuous with the housing base portion 4b on the axially proximal side, and an operating portion 4c that extends from the housing restriction portion 4a toward the axially proximal side. The housing restriction portion 4a can release the catch on the housing caught portion 2b2 by swinging radially outward from the flow passage tubular member 3 due to elastic deformation, in response to a radially inward operation on the operating portion 4c.

The housing 4 has a first housing restriction portion 4a, a first operating portion 4c extending from the first housing restriction portion 4a to the axially proximal side, a second housing restriction portion 4a, and a second operating portion 4c extending from the second housing restriction portion 4a to the axially proximal side. The flow passage tubular member 3 is located between the first housing restriction portion 4a and the second housing restriction portion 4a, or the first housing restriction portion 4a and the second housing restriction portion 4a are provided so as to face each other (offset by 180°).

The flow passage tubular member 3 and the root portion of the housing base portion 4b form an integral member. The housing restriction portion 4a, the portion other than the root portion of the housing base portion 4b, and the operating portion 4c form an integral member.

In the present embodiment, a connection structure 8 includes the male connector 1 and the female connector 2.

The female connector 2 includes an elastic valve body 2a and a housing member 2b, and the housing member 2b forms a female connector flow passage C2 and a connection port P continuous with the female connector flow passage C2 and in which the elastic valve body 2a is disposed. Furthermore, the housing member 2b has a movable body caught portion 2b1 and a housing caught portion 2b2.

The elastic valve body 2a has a top surface 2a1 pressed against the distal surface of the elastic opening/closing portion 5 through the connecting operation, and a slit 2a2 through which the flow passage tubular member 3 passes from the top surface 2a1 when the form changes from the first form to the second form. The elastic valve body 2a is formed of, for example, rubber or elastomer.

Each of the male connector 1 and the female connector 2 is configured as a medical device. The male connector 1 and the female connector 2 can constitute, for example, an infusion set. The infusion set includes, for example, the male connector 1, the female connector 2, an infusion container, a drip chamber, and a medical tube. The fluid is not particularly limited, and is, for example, a medical fluid (such as an infusion agent, a nutrient, or an anticancer agent), a saline solution, or the like.

The embodiments of the present disclosure have been described above. However, the present disclosure is not limited to the above-described embodiments, and thus the above-described embodiments can be variously modified without departing from the gist of the present disclosure.

### Reference Signs List

- 1: Male connector
- 2: Female connector
- 2a: Elastic valve body
- 2a1: Top surface
- 2a2: Slit
- 2b: Housing member
- 2b1: Movable body caught portion
- 2b2: Housing caught portion
- 3: Flow passage tubular member
- 3a: Opening
- 3b: Tapered surface
- 4: Housing
- 4a: Housing restriction portion
- 4a1: Housing catching portion
- 4b: Housing base portion
- 4c: Operating portion
- 5: Elastic opening/closing portion
- 5a: Slit
- 6: Movable body
- 6a: Movable body restriction portion
- 6a1: Receiving surface
- 6a2: Bent portion
- 6a3: Movable body catching portion
- 6a4: Arm portion
- 6a5: Protrusion
- 6b: Holding portion
- 6c: Movable body base portion
- 7: Biasing portion
- 8: Connection structure
- C1: Male connector flow passage
- C2: Female connector flow passage
- S: Hollow portion
- P: Connection port

## Claims

1. A male connector connectable to a female connector having an elastic valve body, the male connector comprising:
a flow passage tubular member defining an axial direction and a radial direction and having an opening at a portion on an axially distal side;
a housing continuous with the flow passage tubular member;
an elastic opening/closing portion;
a movable body having a movable body restriction portion and holding the elastic opening/closing portion; and
a biasing portion including all portions capable of biasing the elastic opening/closing portion toward the axially distal side,
wherein the male connector changes from a first form in which the opening of the flow passage tubular member is closed by the elastic opening/closing portion at an initial position to a second form in which the opening of the flow passage tubular member is opened by a connecting operation where the elastic valve body is pressed against a distal surface of the elastic opening/closing portion against a biasing force of the biasing portion to cause the elastic opening/closing portion and the movable body to move toward an axially proximal side relative to the flow passage tubular member, such that the portion on the axially distal side of the flow passage tubular member protrudes from the elastic opening/closing portion toward the axially distal side upon completion of the connecting operation,
the movable body restriction portion restricts movement of the female connector toward the axially distal side relative to the elastic opening/closing portion by moving radially inward upon receiving a reaction force from the housing due to the connecting operation, and releases the restriction on the movement of the female connector by moving radially outward through a release operation that changes a form from the second form to the first form, and
the biasing force becomes maximum after start of the connecting operation and before completion of the connecting operation.

2. The male connector according to claim 1, wherein the portion on the axially distal side of the flow passage tubular member has a tapered surface in which an outer peripheral surface narrows toward the axially distal side,
in the first form, the elastic opening/closing portion is in contact with the tapered surface, and
the biasing portion is constituted by the tapered surface.

3. The male connector according to claim 2, wherein the tapered surface of the flow passage tubular member extends across the opening in the axial direction.

4. The male connector according to claim 1, wherein the movable body restriction portion has a receiving surface that receives a reaction force from the housing toward the axially distal side by being pressed against the housing radially outward by a restoring force due to elastic deformation of the movable body restriction portion, and
the biasing portion is constituted by the receiving surface.

5. The male connector according to claim 1, wherein the movable body restriction portion is caught on the female connector to restrict the movement of the female connector toward the axially distal side relative to the elastic opening/closing portion.

6. The male connector according to claim 1, wherein the housing has a housing restriction portion that restricts the movement of the female connector toward the axially distal side relative to the elastic opening/closing portion by changing a form from the first form to the second form.

7. The male connector according to claim 6, wherein the housing restriction portion is caught on the female connector to restrict the movement of the female connector toward the axially distal side relative to the elastic opening/closing portion.

8. The male connector according to claim 1, configured as a medical device.

9. A connection structure comprising: the male connector according to claim 1; and the female connector.

10. The connection structure according to claim 9, wherein each of the male connector and the female connector is configured as a medical device.
